# EUROPÄISCHE PATENTSCHRIFT

(11) **EP 2 057 459 B1**
(45) Veröffentlichungstag und Bekanntmachung des Hinweises auf die Patenterteilung: **13.03.2013**
(21) Anmeldenummer: 07786497.3
(22) Anmeldetag: 01.08.2007
(51) Int. Cl.: G01N 21/64, C02F 1/00

(54) **VERFAHREN UND VORRICHTUNG ZUR DETEKTION LEBENDER PHYTOPLANKTONZELLEN IN WASSER**
METHOD AND APPARATUS FOR THE DETECTION OF LIVING PHYTOPLANKTON CELLS IN WATER
PROCÉDÉ ET DISPOSITIF DE DÉTECTION DE CELLULES DE PHYTOPLANCTON VIVANTES DANS L'EAU

(30) Priorität: 01.09.2006 DE 102006041347
(43) Veröffentlichungstag der Anmeldung: 13.05.2009
(73) Patentinhaber: RWO GmbH, 28307 Bremen (DE)
(72) Erfinder: KROON, Bernd, 27607 Langen (DE); KORNMÜLLER, Anja, 28213 Bremen (DE)
(74) Vertreter: Methling, Frank-Oliver
(86) Internationale Anmeldenummer: PCT/EP2007/006812
(87) Internationale Veröffentlichungsnummer: WO 2008/025428

(56) Entgegenhaltungen:
- EP-A- 1 582 859
- DE-A1- 4 427 438
- DE-A1- 19 930 865
- JP-A- 61 071 339
- US-A- 5 480 562
- MOLDAENKE C ET AL: "The 1-Hz fluorometer: A new approach to fast and sensitive long-term studies of active chlorophyll and environmental influences" HELGOLAENDER MEERESUNTERSUCHUNGEN, Bd. 49, Nr. 1-4, 1995, Seiten 785-796, XP008085547 ISSN: 0174-3597
- PARESYS G ET AL: "Quantitative and qualitative evaluation of phytoplankton communities by trichromatic chlorophyll fluorescence excitation with special focus on cyanobacteria" WATER RESEARCH, ELSEVIER, AMSTERDAM, NL, Bd. 39, Nr. 5, März 2005 (2005-03), Seiten 911-921, XP004789874 ISSN: 0043-1354
- DUBINSKY ZVY ET AL: "Listening to phytoplankton: Measuring biomass and photosynthesis by photoacoustics" JOURNAL OF PHYCOLOGY, Bd. 34, Nr. 5, Oktober 1998 (1998-10), Seiten 888-892, XP002457528 ISSN: 0022-3646

## Beschreibung

Die Erfindung betrifft ein Verfahren und eine Vorrichtung zur Detektion lebender Phytoplanktonzellen in/aus Wasser, insbesondere Oberflächenwasser wie Bäche, Flüsse, Teiche, Seen und Talsperren, Süß-, Meer- und Brackwasser, Ballastwasser von Schiffen, Tiefseewasser, Grund- und Quellwasser, Prozess-, Industrie-, Kühl- und Kreislaufwasser, Abwasser, Bade- und Schwimmwasser, Zuchtwasser und Zuchtmedien oder Produktionswasser.

Bei der Nutzung von natürlichen Wässern ist die Entfernung von Phytoplankton und/oder Mikroorganismen in der Wasseraufbereitung ein wichtiges Ziel, um das Wasser für verschiedene Zwecke nutzbar zu machen und Aufbereitungs- und Einleitgrenzwerte einzuhalten. Zum Beispiel ist die Massenentwicklung von Phytoplankton in Oberflächengewässern und deren Durchbruch durch die Filteranlagen und Auftreten im Trinkwasserverteilungsnetz ein bekanntes Problem. Bei ungenügender Aufbereitung entstehen Probleme durch Phytoplankton selbst, wie z. B. eine Färbung des Wassers, eine Abgabe von Geruchsstoffen und Toxinen sowie die Vermehrung anderer unerwünschter Bakterien im Wasser, welche wiederum Phytoplankton als Nahrungsgrundlage nutzen. Gleichzeitig ist eine Aussetzung und/oder Verschleppung von Phytoplanktonarten in artfremde Biotope unerwünscht, weil hierdurch eine Verschiebung im ökologischen Gleichgewicht verursacht wird.

Es ist daher erforderlich, lebende Phytoplanktonzellen in Wasser zu detektieren, insbesondere im Rahmen von Überwachungs- und Steuerungsverfahren online zu detektieren, insbesondere zur Steuerung eines Behandlungsverfahrens zur Entfernung und/oder Desinfektion der lebenden Phytoplanktonzellen und/oder Mikroorganismen in dem Wasser.

Die heute bekannten Methoden sind jedoch gerade in Grenzbereichen bei Zellgrößen kleiner oder gleich 0,1 mm und bei der notwendigen Bestimmung der Zellzahl nicht in der Lage, Ergebnisse zuverlässig und innerhalb sinnvoller Zeitgrenzen, insbesondere bei Vorhandensein unterschiedlicher Arten und beliebiger Zusammenstellung, zu liefern. So ist das bekannte Verfahren der Vermehrung der Biomasse zwar sehr sensitiv, jedoch sehr zeitaufwendig, da es Tage oder gar Wochen bedarf, um die Biomasse zu bestimmen. Weiterhin nachteilig bei diesem Verfahren ist, dass die ursprüngliche Zellzahl unbekannt bleibt. Daher ist diese Methode nicht zur Online-Überwachung geeignet.

Weiterhin bekannt ist die passive fluorometrische Methode, mit der man die Biomasse der Phytoplanktonzellen in einer Wasserprobe bestimmen kann. Nachteilig bei dieser Methode ist, dass sie keinen Aufschluss über lebende oder tote Phytoplanktonzellen liefert, da keine Unterscheidung möglich ist.

Die sogenannte aktive fluorometrische Methode dient zur Bestimmung der Quantumseffizienz des Photosynthesesystems, welche spezifisch nur für lebende Zellen bestimmt werden kann. Nachteilig bei dieser Methode ist, dass sie keine Quantifizierung von Phytoplanktonzellen erlaubt.

Aus der DE 199 30 865 A1 ist ein Chlorophyllfluorometer zur Phytoplanktonbestimmung bekannt, bei dem eine Wasserprobe in einem zweigeteilten Probenraum untersucht wird, wobei der eine Teil einem phototaktisch unwirksamen Fluoreszenz-Meßlicht und der andere Teil mit einem phototaktisch wirksamen Licht belichtet wird, welches nur bei Dinoflagellaten typische Bewegungen zwischen den beiden Teilvolumina auslöst, wobei durch die dadurch bedingten zeitabhängigen Fluoreszenzänderungen für bestimmte Dinoflagellaten charakteristisch sind. Hierdurch können Informationen hinsichtlich der Artenzusammensetzung der Dinoflagellaten bei unbekannten Wasserproben gewonnen werden, nachteilig ist jedoch, dass keine quantitativen Aussagen über die absolute Anzahl lebender Zellen einer bestimmten Spezies in der Wasserprobe gewonnen werden können.

Aus MOLDAENKE, C. et al.: "The 1-Hz fluorometer: A new approach to fast and sensitive long-term studies of active chlorophyll and environmental influences", HELGOLÄNDER MESSUNTERSUCHUNGEN, Bd. 49, Nr. 1-4, 1995, Seiten 785-796. XPO08085547 ISSN: 0174-3597 ist ein Verfahren zur quantitativen Bestimmung der konzentration von lebenden Phytoplanktonzellen in einer Wasserprobe bekannt, bei dem die durch ein moduliertes aktinisches Licht verursachte Modulation der Fluoreszenz von Phytoplanktonzellen ermittelt wird. Diese Modulation entspricht der Modulation des "redox"-Zustands der primären Akzeptoren des Chlorophylls, und erfolgt nur bei lebenden Phytoplanktonzellen. Dieses Verfahren wird mit einer Probe kalibriert, die die gleiche Spezies von Zellen wie in der Wasserprobe beinhaltet, um die Anzahl von lebenden Phytoplanktonzellen in der Wasserprobe zu ergeben.

PARESYS, G. et al.: "Quantitative and qualitative evaluation of phytoplankton communities by trichromatic chlorophyll fluorescence excitation with special focus on cyanobacteria", WATER RESEARCH, ELSEVIER, AMSTERDAM, NL, Bd. 39, Nr. 5, März 2005 (2005-03), Seiten 911-921, XP004789874 ISSN: 0043-1354 beschreibt ein Verfahren zur Bestimmung der Konzentration bekannter Referenzorganismen auf der Basis der Messung der minimalen Fluoreszenz. Nachteilig ist dabei, dass dieses Verfahren nur anwendbar ist auf bekannte Spezies, wobei nur lebende Zellen dieser bekannten Spezies Berücksichtigung finden. Eine Unterscheidung zwischen lebenden und toten Zellen ist nicht möglich. Auch ist es mit diesem Verfahren nicht möglich, die Anzahl lebender Zellen einer unbekannten Spezies in einer Wasserprobe zu bestimmen. Ferner eignet sich dieses Verfahren nicht für eine Online-Diagnose.

Aus der EP 1 582 859 A1 ist ein Verfahren zur Bestimmung von Mikroben bekannt, bei dem die Fluoreszenz der von Sporen gebildeten Farbstoffen gemessen wird. Dieses Verfahren ist jedoch nicht geeignet, die Anzahl lebender Phytoplanktonzellen in einer Wasserprobe zu bestimmen.

DUBINSKY, ZVY et al.: "Listening to phytoplankton: Measuring biomass and phytosynthesis by photoacoustics", JOURNAL OF PHYCOLOGY, Bd. 34, Nr. 5, Oktober 1998 (1998-10), Seiten 888-892, XP002457528 ISSN: 0022-3646 beschreibt ein Verfahren auf Basis der Messung der Wärmeentwicklung infolge eines Lichteintrags.

Aufgabe der Erfindung ist es, ein Verfahren und eine Vorrichtung zur Detektion lebender Phytoplanktonzellen in/aus Wasser zu schaffen, mit der es möglich ist, die Anzahl lebender Phytoplanktonzellen in einer Wasserprobe mit geringem Aufwand und in kürzester Zeit zu bestimmen und insbesondere eine Online-Überwachung von Wasser zu ermöglichen.

Diese Aufgabe wird durch ein Verfahren gemäß Anspruch 1 sowie eine Vorrichtung gemäß Anspruch 17 gelöst.

Das erfindungsgemäße Verfahren zur Detektion lebender Phytoplanktonzellen in oder aus Wasser weist die folgenden Schritte auf:
● Messung von minimaler Fluoreszenz (Fo) entsprechend einem Zustand, bei dem praktisch alle primären Elektronenakzeptoren noch oxidiert sind, entsprechend einem Zustand bei Dunkelheit oder durch Anwendung einer Lichtquelle eines Lichts mit über 700 nm, ● Messung der maximalen Fluoreszenz (Fm) bei einem Eintrag von Licht, entsprechend der Fluoreszenz, bei der zumindest annähernd alle primären Elektronenakzeptoren reduziert sind, sowie
● Berechnung der variablen Fluoreszenz Fv durch Bildung der Differenz von maximaler Fluoreszenz Fm minus minimaler Fluoreszenz Fo in einem Messraum, unabhängig von seiner Geometrie, der das zu prüfende Wasser und/oder Organismen enthält, und
● Berechnung der Anzahl lebender Phytoplanktonzellen einer Referenzart in dem Messraum in Abhängigkeit der variablen Fluoreszenz Fv.

Erfindungsgszenmäβ erfolgt eine Berechnung einer äquivalenten Anzahl lebender Phytoplanktonzellen anderer Zellgrößen als der Referenzart, insbesondere durch volumetrischen Vergleich. Die Zellinhalte von Phytoplanktonzellen sind üblicherweise proportional zu dem Volumen der Zellen. Anhand dieses Zusammenhanges kann eine Berechnung einer äquivalenten Anzahl lebender Phytoplanktonzellen bei abweichenden Zellgrößen als der Zellgröße der Referenzart erfolgen.

Dabei bezeichnet die minimale Fluoreszenz Fo die Fluoreszenz aus lebenden und toten Zellen, die maximale Fluoreszenz Fm entspricht der Fluoreszenz, bei der zumindest annähernd alle primären Elektronenakzeptoren reduziert sind, und die variable Fluoreszenz Fv entspricht der Differenz zwischen der maximalen Fluoreszenz Fm und der minimalen Fluoreszenz Fo, jeweils bezogen auf das in dem Messraum befindliche Wasser und/oder Organismen, welches zu prüfen ist.

Zur Ermittlung biologischen Materials im Wasser kann mittels eines Fluorometers die Fluoreszenz erfasst werden. Dabei können zwei Zustände unterschieden werden, zum einen die minimale Fluoreszenz Fo (dunkler Zustand) sowie die maximale Fluoreszenz Fm bei einem Eintrag von Licht, insbesondere von Licht vorgegebener Wellenlänge. Es hat sich überraschend gezeigt, dass die Differenz von maximaler Fluoreszenz Fm minus der minimalen Fluoreszenz Fo, d. h. die variable Fluoreszenz Fv, ein Maß für die Anzahl lebender Phytoplanktonzellen in dem Messraum bzw. der Prüfmenge des Wassers und/oder der Organismen ist, da die variable Fluoreszenz Fv und die Anzahl lebender Zellen korrelieren.

Durch eine Messung von minimaler Fluoreszenz Fo (ohne Beleuchtung), eine Messung der maximalen Fluoreszenz Fm (bei Beleuchtung) sowie die Berechnung der variablen Fluoreszenz Fv durch Bildung der Differenz Fm minus Fo, kann die Anzahl lebender Phytoplanktonzellen einer Referenzart in dem Messraum bzw. der Prüfmenge des Wassers und/oder der Organismen berechnet werden.

Bei der erfindungsgemäßen Vorrichtung zur Detektion lebender Phytoplanktonzellen in/aus Wasser ist zumindest ein Fluorometer zur Ermittlung der minimalen Fluoreszenz Fo und der maximalen Fluoreszenz Fm einer Wassermenge und/oder Organismenmenge innerhalb eines Prüfraumes vorgesehen, wobei das Fluorometer wehrere Lichtquellen und zumindest einen Detektor aufweist. Des weiteren ist eine Auswerteeinheit vorgesehen, mittels derer eine Ermittlung der variablen Fluoreszenz Fv sowie eine Berechnung der Anzahl lebender Phytoplanktonzellen einer Referenzart im Testvolumen in Abhängigkeit der ermittelten variablen Fluoreszenz Fv erfolgt und eine Berechnung einer äquivalenten Anzahl lebender Phytoplanktonzellen anderer Zellgrößen als der Referenzart mittels eines volumetrischen Vergleichs, erfolgt,

Wobei eine Stenereinheit vorgesehen ist, mittels derer eine Entfernungs- und/oder Desinfektionseinrichtung steuerbar ist, wobei der Vorrichtung eine Entfernungs- und/oder Desinfektionseinrichtung vor- oder nachgeschaltet ist, die ihre Steuerbefehle von der Steuereinheit erhält.

Die Intensität des fluoreszierenden Lichtes ist direkt proportional zur Anzahl der Zellen einer Referenzart in dem Messraum respektive der Prüfmenge in/aus dem Wasser, d. h. der Zusammenhang folgt einer Geraden, wobei die Steigung der Proportionalitätsgeraden wiederum ein Maß für die Größe der einzelnen Zellen ist.

Bei dem "Prüfraum" kann es sich um ein Testvolumen handeln, welches mit dem zu untersuchenden Wasser gefüllt ist, d. h. eine Wasserprobe, es kann sich jedoch auch um einen Membranfilter handeln, mittels dessen eine bestimmte Menge des zu untersuchenden Wassers filtriert wurde und wobei die Messung der minimalen Fluoreszenz Fo und der maximalen Fluoreszenz Fm direkt mit der Zellschicht auf der Fläche des Membranfilters ohne Wasser erfolgt.

Es hat sich überraschend gezeigt, dass die Ermittlung der Anzahl lebender Zellen auf der Basis der Berechnung der variablen Fluoreszenz Fv durch die lebenden Zellen infolge eines Lichteintrags, d.h. einer kurzzeitigen Bestrahlung der Zellen in einem Messraum mit Licht, möglich ist.

Die variable Fluoreszenz Fv ist somit ein Maß für die Anzahl der lebenden Zellen in dem Messvolumen, d.h. die beiden Größen sind insbesondere untereinander äquivalent, da lebende Zellen infolge einer kurzzeitigen Bestrahlung mit Licht Fluoreszenz entwickeln und somit eine Berechnung der Anzahl lebender Zellen einer Referenzart in dem Messraum in Abhängigkeit der variablen Fluoreszenz möglich ist.

Weitere vorteilhafte Ausgestaltungen der Erfindung sind in den Unteransprüchen angegeben.

So ist es vorteilhaft, wenn die lineare Kalibrierung zur Ermittlung des Zusammenhanges zwischen variabler Fluoreszenz Fv und der Anzahl lebender Phytoplanktonzellen einer Referenzart in dem Messraum ein- order mehrmalig vor der Messung vor der Ermittlung der Fluoreszenz Fo, Fm erfolgt, insbesondere eine Referenzart einer Zellgröße von mehr als 0,8 µm in der kleinsten Länge,

Die Referenzart sowie die Zellgröße von mehr als 0,8 µm in der kleinsten Länge kann mittels bekannter mikroskopischer Verfahren bestimmt bzw. ermittelt werden, d. h. die Referenzart ist vorgebbar.

Anhand dieser linearen Kalibrierung kann nun die Zellzahl aus dem ermittelten Wert der variablen Fluoreszenz Fv berechnet werden.

Bevorzugt erfolgt eine Ermittlung des Streulichtes, insbesondere vor der Ermittlung der minimalen Fluoreszenz Fo und/oder der maximalen Fluoreszenz Fm. Die Ermittlung des Streulichtes kann unmittelbar vor der Durchführung der Messung, insbesondere 50 µs bis 100 µs, insbesondere 80 µs vor der Ermittlung der minimalen Fluoreszenz Fo und/oder der Gesamtfluoreszenz Fm erfolgen. Diese Messung erfasst eventuell anwesendes Streulicht oder jede andere Form von Licht, welche nicht von lebenden Zellen emittiert wird.

Vorzugsweise erfolgt die Ermittlung der minimalen Fluoreszenz Fo durch eine Mittelwertbildung aus mehreren Einzelmessungen der minimalen Fluoreszenz innerhalb des Testvolumens. Bevorzugt erfolgen mehrere Einzelmessungen in einer zeitlichen Folge von 20 ms bis 100 ms.

Alternativ oder kumulativ kann die Ermittlung der maximalen Fluoreszenz Fm durch eine Mittelwertbildung aus mehreren Einzelmessungen der maximalen Fluoreszenz Fm erfolgen, insbesondere können mehrere Einzelmessungen im zeitlichen Abstand von 20 ms bis 100 ms erfolgen.

Durch eine Mittelwertbildung von minimaler Fluoreszenz Fo und/oder maximaler Fluoreszenz Fm kann die Genauigkeit der Messung erhöht werden. Da mehrere Einzelmessungen in sehr kurzer zeitlicher Folge durchgeführt werden können, resultiert hieraus keine relevante Zeitverzögerung bei der Anwendung des Verfahrens, insbesondere im Rahmen einer Online-Überwachung einer Einrichtung zur Überwachung der Qualität des Wassers.

Vorteilhaft ist, wenn die Berechnung der variablen Fluoreszenz Fv unter Verwendung eines Mittelwertes der minimalen Fluoreszenz Fo und/oder eines Mittelwertes der maximalen Fluoreszenz Fm erfolgt. Hierdurch kann ebenfalls die Qualität und Genauigkeit erhöht werden.

Bevorzugt erfolgt die Ermittlung der minimalen Fluoreszenz Fo und der maximalen Fluoreszenz Fm mittels eines Fluorometers unter Einsatz zumindest einer pulsierenden Lichtquelle PL und/oder zumindest einer kontinuierlichen Lichtquelle KL, wobei als Lichtquelle PL und KL insbesondere LEDs dienen. Bevorzugt erfolgt die Ermittlung der minimalen Fluoreszenz Fo unter Verwendung von pulsierendem Licht, insbesondere mit einer Wellenlänge von 420 nm.

Bevorzugt kann das Erreichen des Zustandes zur Ermittlung der minimalen Fluoreszenz Fo beschleunigt werden durch die Anwendung einer Lichtquelle mit einer Wellenlänge größer 700 nm, wobei als Lichtquelle insbesondere LEDs dienen.

Bevorzugt erfolgt die Ermittlung der minimalen Fluoreszenz Fo unter Verwendung von zumindest einer pulsierenden Lichtquelle PL, insbesondere von Lichtpulsen der pulsierenden Lichtquelle PL mit einem zeitlichen Abstand von 20 ms bis 100 ms.

Die Ermittlung der maximalen Fluoreszenz Fm erfolgt vorzugsweise unter Verwendung von kontinuierlichem Licht, insbesondere mit einer Wellenlänge von 660 nm.

Vorteilhaft ist, wenn die Ermittlung der Fluoreszenz unter Verwendung von zumindest einer pulsierenden Lichtquelle PL, insbesondere von Lichtpulsen der pulsierenden Lichtquelle PL mit einem zeitlichen Abstand von 20 ms bis 100 ms, und zumindest einer kontinuierlichen Lichtquelle KL erfolgt.

Im Rahmen einer kontinuierlichen Überwachung kann das Verfahren, d. h. die einzelnen Verfahrensschritte in vorgebbarer Anzahl wiederholt werden.
Hierdurch ist es möglich, eine kontinuierliche Überwachung und Qualitätssicherung des zu prüfenden Wassers durchzuführen und im Rahmen einer Online-Überwachung zu realisieren. Insbesondere kann durch eine Überwachung eines vorgegebenen Grenzwertes automatisch ein Alarm ausgelöst werden.

Vorteilhaft ist es, wenn in einem kontinuierlichen Überwachungsprozess aus einem Wasservorrat und/oder einem Wasserstrom wiederholt Testvolumina oder Testmengen entnommen werden und die Verfahrensschritte jeweils ein oder mehrfach auf jedes Testvolumen angewandt werden und die jeweils berechnete Anzahl lebender Phytoplanktonzellen einer Überwachungs- und/oder Steuerungseinheit übermittelt werden. Insbesondere können die ermittelten Daten, d. h. berechnete Anzahl lebender Phytoplanktonzellen in dem Messraum einer Einrichtung zugeführt werden, mit der die Entfernung von Zellen aus dem Wasser und/oder die Desinfektion des Wassers gesteuert und/oder überwacht wird.

Eine Steuerung eines Behandlungsverfahrens zur Entfernung und/oder Desinfektion der lebenden Phytoplanktonzellen in/aus dem Wasser in Abhängigkeit der berechneten Anzahl lebender Phytoplanktonzellen und/oder in Abhängigkeit einer berechneten äquivalenten Anzahl lebender Phytoplanktonzellen kann somit erfolgen.

Vorzugsweise erfolgt eine flüchtige oder dauerhafte Speicherung zumindest der berechneten Anzahl der lebenden Phytoplanktonzellen insbesondere zu Dokumentationszwecken.

Vorteilhaft ist es, wenn die berechnete Anzahl der lebenden Phytoplanktonzellen in dem Wasser auf Überschreitung eines vorgebbaren Grenzwertes überwacht wird, insbesondere dass bei Überschreitung des Grenzwertes eine Alarmauslösung erfolgt.

Die erfindungsgemäße Vorrichtung zur Detektion lebender Phytoplanktonzellen in/aus Wasser weist vorzugsweise einen Messraum auf, der insbesondere durch eine Küvette gebildet ist, insbesondere eine Küvette aus Glas oder Kunststoff bestehend.

Bevorzugt weist der Prüfraum einen Einlass und/oder einen Auslass auf. Insbesondere kann der Prüfraum in einen kontinuierlichen oder getakteten Förderstrom des zu prüfenden Wassers eingebettet, d. h. mittels des Einlasses und des Auslasses in eine Förderleitung integriert sein.

Vorzugsweise weist die Vorrichtung zumindest eine pulsierende Lichtquelle und/oder zumindest eine kontinuierliche Lichtquelle auf, wobei als Lichtquelle insbesondere LEDs dienen.

Vorzugsweise sind zumindest eine Lichtquelle pulsierenden Lichts, insbesondere blauen Lichts mit einer Wellenlänge von etwa 420 nm, und zumindest eine Lichtquelle kontinuierlichen Lichts, insbesondere roten Lichts mit einer Wellenlänge von etwa 660 nm und eine Lichtquelle mit einer Wellenlänge größer 700 nm, augeorduet.

Bevorzugt ist der Vorrichtung eine Einrichtung zur Entfernung von Zellen aus dem Wasser und/oder zur Desinfektion des Wassers vor- und/oder nachgeschaltet.

Bevorzugt ist zumindest ein steuerbares Ventil an einem Einlass und/oder an einem Auslass des Prüfraumes angeordnet. Hierdurch ist es möglich, die Vorrichtung in einen kontinuierlichen Förderprozess einzubinden, wobei die Förderung des zu prüfenden Wassers mittels des steuerbaren Ventils für die Zeit einer Messung mittels des Fluorometers unterbrochen werden kann.

Vorteilhaft ist die Anordnung einer Förderpumpe zur Förderung des Wassers.

Bevorzugt ist eine Steuereinheit vorgesehen, mittels derer die Auswerteeinheit und/oder eines oder mehrerer Ventile und/oder eine Förderpumpe und/oder eine Entfernungs- und/oder Desinfektionseinrichtung steuerbar ist.

Vorteilhaft ist, wenn eine Speichereinheit vorgesehen ist, mittels derer zumindest die ermittelte variable Fluoreszenz Fv und/oder die berechnete Anzahl lebender Phytoplanktonzellen und/oder Mikroorganismen flüchtig oder dauerhaft speicherbar ist.

Hierdurch wird eine überprüfbare Dokumentation ermöglicht.

Die Erfindung wird anhand der Figuren näher erläutert. Es zeigen:
- Fig. 1: ein Beispiel einer Fluoreszenzinduktionskurve;
- Fig. 2: ein Schema eines Ausführungsbeispieles der Vorrichtung zur Detektion lebender Phytoplanktonzellen und/oder Mikroorganismen in Wasser;
- Fig. 3: den Zusammenhang zwischen der variablen Fluoreszenz Fv und der Anzahl der lebenden Zellen pro Milliliter in einem Messvolumen.

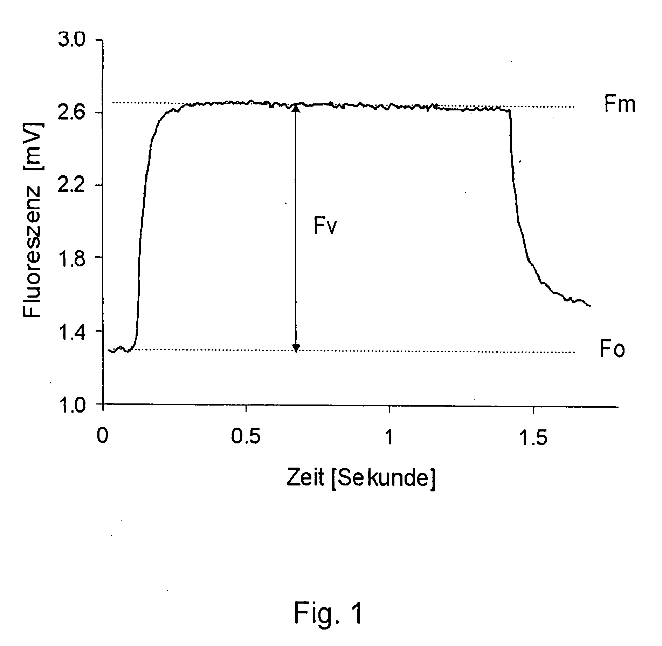

Fig. 1 zeigt eine gemessene Fluoreszenzinduktionskurve im zeitlichen Verlauf. Das Niveau minimaler Fluoreszenz Fo wird erreicht, wenn ein Zustand hervorgerufen wird, wo praktisch alle primären Elektronakzeptoren noch oxidiert sind, entsprechend einem Zustand bei Dunkelheit oder durch Anwendung einer Lichtquelle eines Lichts mit einer Wellenlänge von über 700 nm. Durch Einschalten einer intermittierenden oder kontinuierlichen Lichtquelle zum Zeitpunkt T1 werden fotochemische Reaktionen aktiviert, die zur Folge haben, dass die primären Elektronakzeptoren reduziert werden. Wenn zumindest annähernd alle primären Elektronakzeptoren reduziert sind, erreicht das Fluoreszenzniveau die maximale Fluoreszenz Fm. Dem Verlauf der Fluoreszenzinduktionskurve über der Zeit nach Einschalten der Lichtquelle zum Zeitpunkt T1 ist entnehmbar, dass ein Anstieg der Fluoreszenz von der minimalen Fluoreszenz Fo auf die maximale Fluoreszenz Fm nicht sprunghaft erfolgt, sondern in einem dynamischen Prozess kontinuierlich ansteigt, was auf das Verhalten der lebenden Phytoplanktonzellen zurückzuführen ist.

Der Abfall der Fluoreszenzinduktionskurve nach einem Abschalten der Lichtquelle zum Zeitpunkt T2 ist ebenfalls Fig. 1 entnehmbar.

Die variable Fluoreszenz Fv, die der Differenz von maximaler Fluoreszenz Fm minus minimaler Fluoreszenz Fo entspricht, ist proportional zur Anzahl der lebenden Zellen innerhalb des Messraums Anhand eines zuvor, im Rahmen einer Kalibrierung festgestellten Zusammenhanges zwischen variabler Fluoreszenz Fv und der Anzahl lebender Phytoplanktonzellen einer bestimmten vorgegebenen Referenzart, ist es somit möglich, in Abhängigkeit der variablen Fluoreszenz Fv die Anzahl lebender Phytoplanktonzellen der Referenzart zu berechnen.

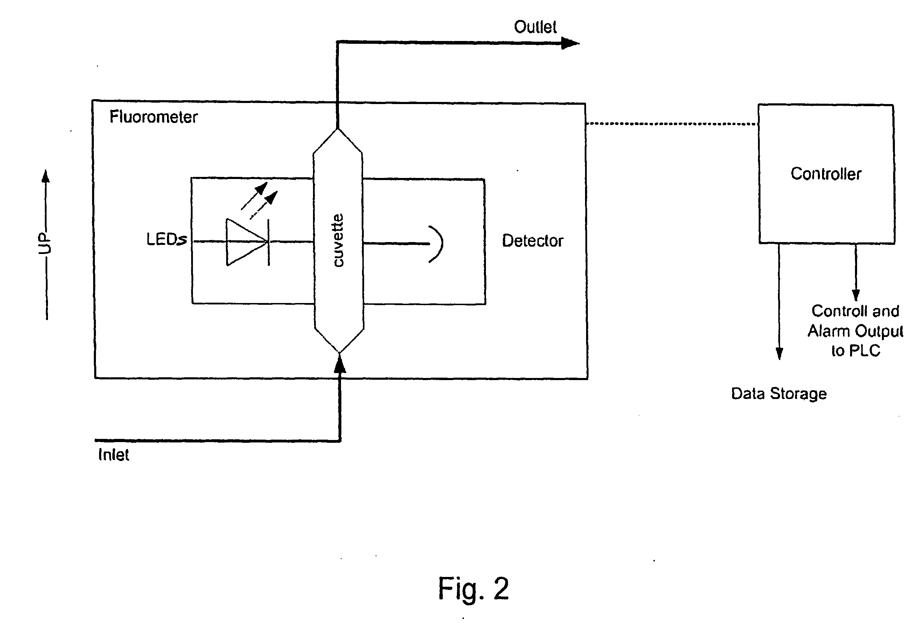

In Fig. 2 ist ein Schema eines Ausführungsbeispieles einer Vorrichtung zur Detektion lebender Phytoplanktonzellen in/aus Wasser dargestellt.

Die Vorrichtung weist ein Fluorometer sowie eine Auswerte-, Überwachungs- und Steuerungseinheit auf.

Der Prüfraum 11 des Fluorometers ist durch eine Küvette mit zwei planparallelen Glasflächen gebildet.

Das Fluorometer weist Lichtquellen in Form von drei Arten von LEDs, eine Lichtquelle emittierend Licht einer Wellenlänge von über 700 nm, eine pulsierende und eine kontinuierliche Lichtquelle auf. Des weiteren weist das Fluorometer einen Detektor auf. Die Lichtquelle wird durch die Steuerungseinheit gesteuert, d. h. ein- und ausgeschaltet. Mittels des Detektors ist es möglich, die Fluoreszenzinduktionskurve der Fluoreszenz der in der Küvette befindlichen und zu prüfenden Wassermenge zu erfassen, d. h. insbesondere bei ausgeschalteter Lichtquelle die minimale Fluoreszenz Fo sowie bei eingeschalteter Lichtquelle die maximale Fluoreszenz Fm zu erfassen und diese an die Steuereinheit zur weiteren Auswertung zu übermitteln.

Das Fluorometer ist eingebunden in einen diskontinuierlichen oder kontinuierlichen Förderprozess und weist einen Einlass und einen Auslass auf.

Mittels der Steuereinheit erfolgt eine Auswertung der über eine Datenleitung vom Fluorometer übermittelten Messwerte der Fluoreszenz, in dem durch Bildung der Differenz von maximaler Fluoreszenz Fm minus minimaler Fluoreszenz Fo die variable Fluoreszenz Fv berechnet wird und aus dieser berechneten variablen Fluoreszenz mittels einer zuvor gemessenen und abgespeicherten Kalibriergerade die Anzahl lebender Phytoplanktonzellen einer Referenzart in dem in der Küvette befindlichen, zu prüfenden Wassers berechnet wird.

Des weiteren erfolgt durch die Steuereinheit eine Überwachung auf Überschreitung eines vorgegebenen und gespeicherten Grenzwertes, wobei bei Überschreitung des Grenzwertes über die Datenleitung eine Alarmmeldung abgegeben wird.

Über eine Datenleitung ist die Steuereinheit mit einer Speichereinheit verbunden, mittels derer die berechnete Anzahl lebender Phytoplanktonzellen speicherbar ist.

Die Vorrichtung ist in einem kontinuierlichen Förderstrom eingebunden und gestattet somit eine kontinuierliche Online-Überwachung des zu prüfenden Wassers.

Der Überwachungsvorrichtung ist eine nicht dargestellte Entfernungs- und/oder Desinfektionseinheit vor- und/oder nachgeschaltet, die ihre Steuerbefehle von der Steuereinheit erhält.

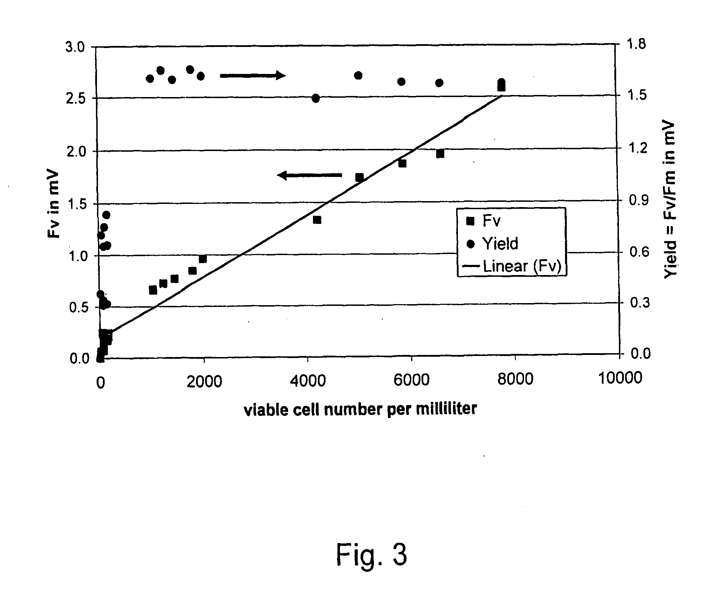

Figur 3 zeigt den Zusammenhang zwischen der variablen Fluoreszenz Fv und der Anzahl der lebenden Zellen pro Milliliter in einem Messvolumen.

Die International Maritime Organization gibt als Ablassstandard von Ballastwasserbehandlungsanlagen an Bord von Schiffen Grenzwerte für gewisse Größenklassen an Organismen vor. Der Größenbereich von ≥ 10 µm bis < 50 µm wird vom Phytoplankton dominiert. Hierfür ist ein Ablassgrenzwert von < 10 lebenden Organismen in der kleinsten Länge pro Milliliter festgeschrieben.

Bisher ist nur eine Überwachung durch langwierige mikroskopische Auszählung an Land möglich. Da die Einleitung des Ballastwassers direkt in die Umwelt erfolgt, ist eine online Überwachung in Echtzeit erforderlich.

In diesem Beispiel gemäß Figur 3 wurde die marine Grünalge *Tetraselmis suecica* mit einer Größe von 10 µm als Referenzorganismus für das Verfahren eingesetzt.

Fig. 3 zeigt die Abhängigkeit des Fv-Signals von der mikroskopisch ausgezählten lebenden Tetraselmis-Zellzahl im Zu- und Ablauf einer Ballastwasserbehandlungsanlage, die aus einer mechanischen Vorabtrennung gefolgt von einer Desinfektionsbehandlung besteht.

Wie die Fig. 3 zeigt, erlaubt der üblicherweise verwendete qualitative Yield (entsprechend Fv/Fm = (Fm-Fo)/Fm) keine Korrelation zur lebenden Zellzahl.

Dagegen ergibt das mit dem erfindungsgemäßen Verfahren bestimmte und weiter verwendete Fv-Signal eine deutliche lineare Abhängigkeit zur Lebendzellzahl (R²= 0.98) auch über einem sehr weiten Zellanzahl-Bereich.

Die Umsetzung dieses Signals für lebende Biomasse zur lebenden Zellanzahl basiert auf einer volumetrischen Beziehung.

Gemäβ der Erfindung werden über die Drittmachtwurzel äquivalente Zellzahlen für andere Zellgrößen als der Referenzart berechnet. Dabei ist die Steigung der Proportionalitätsgeraden ein Maß für die Größe der einzelnen Zellen. Dieser Zusammenhang ist in der nachfolgenden Tabelle wiedergegeben:

| Species | Slope Tetraselmis/Slope Species Steigungsverhältnis | X^{(1/3)} |
|---|---|---|
| Thalassiosira weissflogii | 1.34 | 1.10 |
| Isochrysis galbana | 7.27 | 1.94 |
| Nannochloropsis oculata | 73.80 | 4.19 |

Vorteil der Drittmachtwurzel-Abhängigkeit ist, dass lebende Phytoplanktonzellen in einer Größe kleiner als 10 µm auch in höherer Anzahl das Signal kaum beeinflussen, während lebende Phytoplanktonzellen größer als 10 µm bereits in geringer Zellzahl unter 10 pro Milliliter ein signifikantes Signal verursachen. Dadurch kann eine sichere Überwachung dieses Grenzwertes gewährleistet werden.

## Patentansprüche

1. Verfahren zur Detektion lebender Phytoplanktonzellen in bzw. aus Wasser im Rahmen einer kontinuierlichen Online-Überwachung des Wassers, insbesondere Ballastwasser, Gewässer, Abwässer oder Wasser in Schwimm- oder Badeeinrichtungen, Umfossend die Schritte:
● Messung von minimaler Fluoreszenz (Fo) entsprechend einem Zustand, bei dem praktisch alle primären Elektronenakzeptoren noch oxidiert sind, entsprechend einem Zustand bei Dunkelheit oder durch Anwendung einer Lichtquelle eines Lichts mit über 700 nm,
● Messung der maximalen Fluoreszenz (Fm) bei einem Eintrag von Licht, entsprechend der Fluoreszenz, bei der zumindest annähernd alle primären Elektronenakzeptoren reduziert sind, sowie
● Berechnung der variablen Fluoreszenz (Fv) durch Bildung der Differenz von maximaler Fluoreszenz (Fm) minus minimaler Fluoreszenz (Fo) in einem Messraum und
● Berechnung der Anzahl lebender Phytoplanktonzellen einer Referenzart in dem Messraum in Abhängigkeit der variablen Fluoreszenz (Fv) anhand einer linearen Kalibrierung, wobei eine Berechnung einer äquivalenten Anzahl lebender Phytoplanktonzellen anderer Zellgrößen als der Referenzart mittels eines volumetrischen Vergleichs erfolgt.

2. Verfahren nach Anspruch 1, **dadurch gekennzeichnet, dass** eine lineare Kalibrierung zur Ermittlung des Zusammenhanges zwischen variabler Fluoreszenz (Fv) und der Anzahl lebender Phytoplanktonzellen einer Referenzart in dem Messraum erfolgt, insbesondere einer Referenzart einer Zellgröße von mehr als 0,8 µm in der kleinsten Länge, insbesondere dass die lineare Kalibrierung ein- oder mehrfach vor der Ermittlung der Fluoreszenz (Fo, Fm) erfolgt.

3. Verfahren nach einem der vorherigen Ansprüche, **dadurch gekennzeichnet, dass** eine Ermittlung des Streulichtes erfolgt, insbesondere dass eine Ermittlung des Streulichtes 50 µs bis 100 µs, insbesondere 80 µs, vor der Ermittlung der minimalen Fluoreszenz (Fo) und/oder der maximalen Fluoreszenz (Fm) erfolgt.

4. Verfahren nach einem der vorherigen Ansprüche, **dadurch gekennzeichnet, dass** die Ermittlung der minimalen Fluoreszenz (Fo) durch Mittelwertbildung aus mehreren Einzelmessungen der minimalen Fluoreszenz (Fo) erfolgt, insbesondere dass mehrere Einzelmessungen im Abstand von 20 ms bis 100 ms erfolgen.

5. Verfahren nach einem der vorherigen Ansprüche, **dadurch gekennzeichnet, dass** die Ermittlung der maximale Fluoreszenz (Fm) durch Mittelwertbildung aus mehreren Einzelmessungen der maximalen Fluoreszenz (Fm) erfolgt, insbesondere dass mehrere Einzelmessungen im Abstand von 20 ms bis 100 ms erfolgen.

6. Verfahren nach einem der vorherigen Ansprüche, **dadurch gekennzeichnet, dass** die Berechnung der variablen Fluoreszenz (Fv) unter Verwendung eines Mittelwertes der minimalen Fluoreszenz (Fo) und/oder eines Mittelwertes der maximalen Fluoreszenz (Fm) erfolgt.

7. Verfahren nach einem der vorherigen Ansprüche, **dadurch gekennzeichnet, dass** die Ermittlung der Fluoreszenzen (Fo, Fm) mittels eines Fluorometers unter Einsatz zumindest einer pulsierender Lichtquelle (PL) und/oder zumindest einer kontinuierlicher Lichtquelle (KL) erfolgt, wobei als Lichtquellen (PL, KL) insbesondere LEDs dienen.

8. Verfahren nach einem der vorherigen Ansprüche, **dadurch gekennzeichnet, dass** die Ermittlung der Fluoreszenz unter Verwendung von pulsierendem Licht, insbesondere Licht mit einer Wellenlänge von 420 nm, erfolgt.

9. Verfahren nach einem der vorherigen Ansprüche, **dadurch gekennzeichnet, dass** die Ermittlung der minimalen Fluoreszenz (Fo) unter Verwendung von zumindest einer pulsierenden Lichtquelle (PL) und/oder zumindest einer Lichtquelle mit einer Wellenlänge größer als 700 nm, insbesondere von Lichtpulsen der pulsierenden Lichtquelle (PL) mit einem zeitlichen Abstand von 20 ms bis 100 ms, erfolgt.

10. Verfahren nach einem der vorherigen Ansprüche, **dadurch gekennzeichnet, dass** die Ermittlung der maximalen Fluoreszenz (Fm) unter Verwendung von kontinuierlichem Licht, insbesondere Licht mit einer Wellenlänge von 660 nm, erfolgt.

11. Verfahren nach einem der vorherigen Ansprüche, **dadurch gekennzeichnet, dass** die Ermittlung der maximalen Fluoreszenz (Fm) unter Verwendung von zumindest einer pulsierenden Lichtquelle (PL), insbesondere von Lichtpulsen der pulsierenden Lichtquelle (PL) mit einem zeitlichen Abstand von 20 ms bis 100 ms, und zumindest einer kontinuierlichen Lichtquelle (KL) erfolgt.

12. Verfahren nach einem der vorherigen Ansprüche, **dadurch gekennzeichnet, dass** die Verfahrensschritte in vorgebbarer Anzahl wiederholt werden.

13. Verfahren nach einem der vorherigen Ansprüche, **dadurch gekennzeichnet, dass** in einem kontinuierlichen Überwachungsprozess aus einem Wasservorrat und/oder einem Wasserstrom wiederholt Testvolumina entnommen werden und die Verfahrensschritte jeweils ein- oder mehrfach auf jedes Testvolumen angewandt werden und die jeweils berechnete Anzahl lebender Phytoplanktonzellen einer Überwachungs- und/oder Steuerungseinheit übermittelt werden.

14. Verfahren nach einem der vorherigen Ansprüche, **dadurch gekennzeichnet, dass** eine Steuerung eines Behandlungsverfahrens zur Entfernung und/oder Desinfektion der lebenden Phytoplanktonzellen in dem Wasser in Abhängigkeit der berechneten Anzahl lebender Phytoplanktonzellen und/oder in Abhängigkeit einer berechneten äquivalenten Anzahl lebender Phytoplanktonzellen erfolgt.

15. Verfahren nach einem der vorherigen Ansprüche, **dadurch gekennzeichnet, dass** eine flüchtige oder dauerhafte Speicherung der berechneten Anzahl der lebenden Phytoplanktonzellen in dem Wasser und/oder eine flüchtige oder dauerhafte Speicherung der ermittelten Fluoreszenz/en (Fo, Fm, Fv) erfolgt.

16. Verfahren nach einem der vorherigen Ansprüche, **dadurch gekennzeichnet, dass** die berechnete Anzahl der lebenden Phytoplanktonzellen in dem Wasser auf Überschreitung eines vorgebbaren Grenzwertes überwacht wird, insbesondere dass bei Überschreitung des Grenzwertes eine Alarmmeldung erzeugt wird.

17. Vorrichtung zur Detektion lebender Phytoplanktonzellen in bzw. aus Wasser, mit zumindest einem Fluorometer zur Ermittlung der minimalen Fluoreszenz (Fo) entsprechend einem Zustand, bei dem praktisch alle primären Elektronenakzeptoren noch oxidiert sind, entsprechend einem Zustand bei Dunkelheit oder durch Anwendung einer Lichtquelle eines Lichts mit über 700 nm, und der maximalen Fluoreszenz (Fm) bei einem Eintrag von Licht, entsprechend der Fluoreszenz, bei der zumindest annähernd alle primären Elektronenakzeptoren reduziert sind, innerhalb eines Prüfraumes, wobei das Fluorometer wehrere Lichtquelle und zumindest einen Detektor aufweist, **dadurch gekennzeichnet, dass** eine Auswerteeinheit vorgesehen ist, mittels derer eine Ermittlung der variablen Fluoreszenz (Fv) sowie eine Berechnung der Anzahl lebender Phytoplanktonzellen einer Referenzart in dem Prüfraum in Abhängigkeit der ermittelten variablen Fluoreszenz (Fv) anhand einer linearen Kalibrierung
eine Berechnung einer äquivalenten Anzahl lebender Phytoplanktonzellen anderer Zellgrößen als der Referenzart mittels eines volumetrischen Vergleichs, erfolgt, wobei eine Stenereinheit 7 vorgesehen ist, mittels derer eine Entfernungs- und/oder Desinfektionseinrichtung steuerbar ist, wobei der Vorrichtung eine Entfernungs- und/oder Desinfektionseinrichtung vor- oder nachgeschaltet ist, die ihre Steuerbefehle von der Steuereinheit erhält.

18. Vorrichtung nach Anspruch 17, **dadurch gekennzeichnet, dass** der Prüfraum durch eine Küvette, insbesondere aus Glas oder Kunststoff bestehend, gebildet ist.

19. Vorrichtung nach Anspruch 17 oder 18, **dadurch gekennzeichnet, dass** der Prüfraum einen Einlass und/oder einen Auslass aufweist.

20. Vorrichtung nach einem der Ansprüche 17 bis 19, **dadurch gekennzeichnet, dass** sie zumindest eine pulsierende Lichtquelle und/oder zumindest eine kontinuierliche Lichtquelle aufweist, wobei als Lichtquelle insbesondere LEDs dienen.

21. Vorrichtung nach einem der Ansprüche 17 bis 20, **dadurch gekennzeichnet, dass** zumindest eine Lichtquelle pulsierenden Lichts, insbesondere blauen Lichts mit einer Wellenlänge von etwa 420 nm, und/oder zumindest eine Lichtquelle kontinuierlichen Lichts, insbesondere roten Lichts mit einer Wellenlänge von etwa 660 nm, und/oder eine Lichtquelle mit einer Wellenlänge von mehr als 700 nm, angeordnet sind.

22. Vorrichtung nach einem der Ansprüche 17 bis 21, **dadurch gekennzeichnet, dass** zumindest ein steuerbares Ventil an einem Einlass und/oder an einem Auslass des Prüfraumes angeordnet ist.

23. Vorrichtung nach einem der Ansprüche 17 bis 22, **dadurch gekennzeichnet, dass** eine Förderpumpe zur Förderung des Wassers vorgesehen ist.

24. Vorrichtung nach einem der Ansprüche 17 bis 23, **dadurch gekennzeichnet, dass** eine Steuereinheit vorgesehen ist, mittels derer die Auswerteeinheit und/oder eines oder mehrere Ventile und/oder eine Förderpumpe und/oder steuerbar ist.

25. Vorrichtung nach einem der Ansprüche 17 bis 24, **dadurch gekennzeichnet, dass** eine Speichereinheit vorgesehen ist, mittels derer die gemessenen Fluoreszenz/en (Fo, Fm) und/oder die ermittelte variable Fluoreszenz (Fv) und/oder die berechnete Anzahl lebender Phytoplanktonzellen flüchtig oder dauerhaft speicherbar ist.

## Claims

1. Method for detecting living phytoplankton cells in or from water in the course of continuous online monitoring of the water, in particular ballast water, bodies of water, sewage or water in swimming or bathing facilities, comprising the steps of:
● measuring minimal fluorescence (Fo) corresponding to a state in which almost all primary electron acceptors are still oxidised, corresponding to a state of darkness or using a light source of a light having over 700 nm,
● measuring the maximal fluorescence (Fm) during an input of light, corresponding to the fluorescence in which at least almost all primary electron acceptors are reduced, as well as
● calculating the variable fluorescence (Fv) by forming the difference between maximal fluorescence (Fm) and minimal fluorescence (Fo) in a measuring space, and
● calculating the number of living phytoplankton cells of a reference species in the measuring space on the basis of the variable fluorescence (Fv) by means of a linear calibration, wherein an equivalent number of living phytoplankton cells of cell sizes other than the reference species is calculated by means of a volumetric comparison.

2. Method according to claim 1, **characterised in that** a linear calibration for determining the relationship between variable fluorescence (Fv) and the number of living phytoplankton cells of a reference species is carried out in the measuring space, in particular of a reference species having a cell size of greater than 0.8 µm in the smallest length, in particular **in that** the linear calibration is carried out one or more times before the fluorescence (Fo, Fm) is determined.

3. Method according to any of the preceding claims, **characterised in that** the scattered light is determined, in particular **in that** the scattered light from 50 µs to 100 µs, in particular 80 µs, is determined before the minimal fluorescence (Fo) and/or the maximal fluorescence (Fm) is/are determined.

4. Method according to any of the preceding claims, **characterised in that** the minimal fluorescence (Fo) is determined by averaging from a plurality of individual measurements of the minimal fluorescence (Fo), in particular **in that** a plurality of individual measurements are carried out at intervals of from 20 ms to 100 ms.

5. Method according to any of the preceding claims, **characterised in that** the maximal fluorescence (Fm) is determined by averaging from a plurality of individual measurements of the maximal fluorescence (Fm), in particular **in that** a plurality of individual measurements are carried out at intervals of from 20 ms to 100 ms.

6. Method according to any of the preceding claims, **characterised in that** the variable fluorescence (Fv) is calculated using an average value of the minimal fluorescence (Fo) and/or an average value of the maximal fluorescence (Fm).

7. Method according to any of the preceding claims, **characterised in that** the fluorescences (Fo, Fm) are determined by means of a fluorometer using at least one pulsating light source (PL) and/or at least one continuous light source (KL), LEDs in particular acting as light sources (PL, KL).

8. Method according to any of the preceding claims, **characterised in that** the fluorescence is determined using pulsating light, in particular light having a wavelength of 420 nm.

9. Method according to any of the preceding claims, **characterised in that** the minimal fluorescence (Fo) is determined using at least one pulsating light source (PL) and/or at least one light source having a wavelength greater than 700 nm, in particular using light pulses of the pulsating light source (PL) at time intervals of from 20 ms to 100 ms.

10. Method according to any of the preceding claims, **characterised in that** the maximal fluorescence (Fm) is determined using continuous light, in particular light having a wavelength of 660 nm.

11. Method according to any of the preceding claims, **characterised in that** the maximal fluorescence (Fm) is determined using at least one pulsating light source (PL), in particular using light pulses of the pulsating light source (PL) at time intervals of from 20 ms to 100 ms, and at least one continuous light source (KL).

12. Method according to any of the preceding claims, **characterised in that** the method steps are repeated a pre-determinable number of times.

13. Method according to any of the preceding claims, **characterised in that** test volumes are repeatedly extracted from a water supply and/or a flow of water in a continuous monitoring process and the method steps are each applied one or more times to each test volume and each calculated number of living phytoplankton cells is transmitted to a monitoring and/or control unit.

14. Method according to any of the preceding claims, **characterised in that** a treatment process for removing and/or disinfecting the living phytoplankton cells in the water is controlled on the basis of the calculated number of living phytoplankton cells and/or on the basis of a calculated equivalent number of phytoplankton cells.

15. Method according to any of the preceding claims, **characterised in that** the calculated number of living phytoplankton cells in the water is temporarily or permanently stored and/or the determined fluorescence(s) (Fo, Fm, Fv) is/are temporarily or permanently stored.

16. Method according to any of the preceding claims, **characterised in that** the calculated number of living phytoplankton cells in the water is monitored when said number exceeds a pre-determinable threshold value, in particular **in that** an alert message is generated when said number exceeds the threshold value.

17. Apparatus for detecting living phytoplankton cells in and/or from water, comprising at least one fluorometer for determining the minimal fluorescence (Fo) corresponding to a state in which almost all primary electron acceptors are still oxidised, corresponding to a state of darkness or using a light source of a light having over 700 nm, and the maximal fluorescence (Fm) during an input of light, corresponding to the fluorescence in which at least almost all primary electron acceptors are reduced, inside a test space, the fluorometer comprising a plurality of light sources and at least one detector, **characterised in that** an evaluation unit is provided, by means of which the variable fluorescence (Fv) is determined and the number of living phytoplankton cells of a reference species in the test space is calculated on the basis of the determined variable fluorescence (Fv) by means of a linear calibration and an equivalent number of living phytoplankton cells of cell sizes other than the reference species being calculated by means of a volumetric comparison, a control unit being provided by means of which a removal and/or disinfection device can be controlled, a removal and/or disinfection device being arranged upstream or downstream of the apparatus, said device receiving its control commands from the control unit.

18. Apparatus according to claim 17, **characterised in that** the test space is formed by a cuvette, in particular consisting of glass or plastics material.

19. Apparatus according to either claim 17 or 18, **characterised in that** the test space has an inlet and/or an outlet.

20. Apparatus according to any of claims 17 to 19, **characterised in that** the apparatus comprises at least one pulsating light source and/or at least one continuous light source, LEDs in particular acting as a light source.

21. Apparatus according to any of claims 17 to 20, **characterised in that** at least one light source of pulsating light, in particular blue light having a wavelength of approximately 420 nm, and/or at least one light source of continuous light, in particular red light having a wavelength of approximately 660 nm, and/or a light source having a wavelength of greater than 700 nm are arranged.

22. Apparatus according to any of claims 17 to 21, **characterised in that** at least one controllable valve is arranged at an inlet and/or at an outlet of the test space.

23. Apparatus according to any of claims 17 to 22, **characterised in that** a delivery pump is provided for delivering the water.

24. Apparatus according to any of claims 17 to 23, **characterised in that** a control unit is provided, by means of which the evaluation unit and/or one or more valves and/or a delivery pump and/or can be controlled.

25. Apparatus according to any of claims 17 to 24, **characterised in that** a memory unit is provided, by means of which the measured fluorescence(s) (Fo, Fm) and/or the determined variable fluorescence (Fv) and/or the calculated number of living phytoplankton cells can be temporarily or permanently stored.

## Revendications

1. Procédé de détection de cellules de phytoplancton vivantes dans l'eau dans le cadre d'un contrôle en ligne continu de l'eau, en particulier des eaux de ballast, de cours d'eau, d'eaux usées ou d'eaux d'installations de piscines ou de bains, comprenant les étapes suivantes :
• la mesure de la fluorescence minimale (Fo) correspondant à un état dans lequel pratiquement tous les accepteurs d'électrons primaires sont encore oxydés, ce qui correspond à un état dans l'obscurité ou par application d'une source de lumière émettant de la lumière de plus de 700 nm,
• la mesure de la fluorescence maximale (Fm) lors d'une application de lumière correspondant à la fluorescence, dans laquelle au moins presque tous les accepteurs d'électrons primaires sont réduits, ainsi que
• le calcul de la fluorescence variable (Fv)
par formation de la différence entre la fluorescence maximale (Fm) et la fluorescence minimale (Fo) dans un espace de mesure, et
• le calcul du nombre de cellules de phytoplancton vivantes d'un type de référence dans l'espace de mesure en fonction de la fluorescence variable (Fv) sur la base d'un étalonnage linéaire, dans lequel un calcul d'un nombre équivalent de cellules vivantes du phytoplancton d'autres grandeurs cellulaires que le type de référence se fait au moyen d'une comparaison volumétrique.

2. Procédé selon la revendication 1, **caractérisé en ce qu'**un étalonnage linéaire permettant d'obtenir le rapport entre la fluorescence variable (Fv) et le nombre de cellules vivantes de phytoplancton d'un type de référence se fait dans l'espace de mesure, en particulier d'un type de référence d'une grandeur cellulaire de plus de 0,8 µm dans la longueur la plus petite, en particulier **en ce que** l'étalonnage linéaire se fait une ou plusieurs fois avant la détermination de la fluorescence (Fo, Fm).

3. Procédé selon l'une quelconque des revendications précédentes, **caractérisé en ce qu'**on effectue une détermination de la lumière diffusée, en particulier **en ce que** l'on procède à une détermination de la lumière diffusée 50 µs à 100 µs, en particulier 80 µs avant la détermination de la fluorescence minimale (Fo) et/ou de la fluorescence maximale (Fm).

4. Procédé selon l'une quelconque des revendications précédentes, **caractérisé en ce que** la détermination de la fluorescence minimale (Fo) se fait par formation de la valeur moyenne de plusieurs mesures individuelles de la fluorescence minimale (Fo), en particulier **en ce que** plusieurs mesures individuelles se font à intervalle de temps de 20 ms à 100 ms.

5. Procédé selon l'une quelconque des revendications précédentes, **caractérisé en ce que** la détermination de la fluorescence maximale (Fm) se fait par formation de la valeur moyenne de plusieurs mesures individuelles de fluorescence maximale (Fm), en particulier **en ce que** plusieurs mesures individuelles se font à intervalle de temps de 20 ms à 100 ms.

6. Procédé selon l'une quelconque des revendications précédentes, **caractérisé en ce que** le calcul de la fluorescence variable (Fv) se fait par utilisation d'une valeur moyenne de la fluorescence minimale (Fo) et/ou d'une valeur moyenne de la fluorescence maximale (Fm).

7. Procédé selon l'une quelconque des revendications précédentes, **caractérisé en ce que** la détermination des fluorescences (Fo, Fm) se fait au moyen d'un fluoromètre en utilisant au moins une source de lumière pulsée (PL) et/ou au moins une source de lumière continue (KL), des DEL en particulier servant de sources de lumière (PL, KL).

8. Procédé selon l'une quelconque des revendications précédentes, **caractérisé en ce que** la détermination de la fluorescence se fait par utilisation de lumière pulsée, en particulier de lumière ayant une longueur d'onde de 420 nm.

9. Procédé selon l'une quelconque des revendications précédentes, **caractérisé en ce que** la détermination de la fluorescence minimale (Fo) se fait par utilisation d'au moins une source de lumière pulsée (PL) et/ou d'au moins une source de lumière ayant une longueur d'onde supérieure à 700 nm, en particulier de pulsations lumineuses de la source de lumière pulsée (PL) à intervalle de temps de 20 ms à 100 ms.

10. Procédé selon l'une quelconque des revendications précédentes, **caractérisé en ce que** la détermination de la fluorescence maximale (Fm) se fait par utilisation de lumière continue, en particulier de lumière ayant une longueur d'onde de 660 nm.

11. Procédé selon l'une quelconque des revendications précédentes, **caractérisé en ce que** la détermination de la fluorescence maximale (Fm) se fait par utilisation d'au moins une source de lumière pulsée (PL), en particulier d'impulsions de lumière de la source de lumière pulsée (PL) à intervalle de temps de 20 ms à 100 ms, et d'au moins une source de lumière continue (KL).

12. Procédé selon l'une quelconque des revendications précédentes, **caractérisé en ce que** les étapes du procédé sont répétées un nombre de fois prédéterminable.

13. Procédé selon l'une quelconque des revendications précédentes, **caractérisé en ce que**, dans un procédé de contrôle continu, on prélève de manière répétée des volumes d'essai d'un réservoir d'eau et/ou d'un cours d'eau et on applique les étapes du procédé respectivement une ou plusieurs fois sur chaque volume d'essai et le nombre respectivement calculé de cellules vivantes du phytoplancton est transmis à une unité de contrôle et/ou de commande.

14. Procédé selon l'une quelconque des revendications précédentes, **caractérisé en ce que** l'on effectue une commande d'un procédé de traitement pour éliminer et/ou désinfecter les cellules de phytoplancton vivantes dans l'eau en fonction du nombre calculé de cellules vivantes du phytoplancton et/ou en fonction d'un nombre calculé équivalent de cellules vivantes du phytoplancton.

15. Procédé selon l'une quelconque des revendications précédentes, **caractérisé en ce que** l'on effectue un enregistrement passager ou continu du nombre calculé des cellules vivantes de phytoplancton dans l'eau et/ou un enregistrement passager ou durable de la ou des fluorescences déterminées (Fo, Fm, Fv).

16. Procédé selon l'une quelconque des revendications précédentes, **caractérisé en ce que** le nombre calculé des cellules vivantes de phytoplancton dans l'eau est contrôlé en matière de dépassement d'une valeur limite prédéterminable, en particulier **en ce que**, lors d'un dépassement de la valeur limite, un message d'alerte est produit.

17. Dispositif de détection de cellules de phytoplancton vivantes dans l'eau, avec au moins un fluoromètre pour déterminer la fluorescence minimale (Fo) correspondant à un état, dans lequel pratiquement tous les accepteurs d'électrons primaires sont encore oxydés, correspondant à un état dans l'obscurité ou par application d'une source de lumière émettant une lumière de plus de 700 nm, et la fluorescence maximale (Fm) pour une application de lumière, correspondant à la fluorescence, dans laquelle au moins presque tous les accepteurs d'électrons primaires sont réduits, à l'intérieur d'un espace d'essai, dans lequel le fluoromètre réduits, à l'intérieur d'un espace d'essai, dans lequel le fluoromètre présente plusieurs sources de lumière et au moins un détecteur, **caractérisé en ce qu'**il est prévu une unité d'évaluation, par laquelle il se fait une détermination de la fluorescence variable (Fv) ainsi qu'un calcul de la quantité de cellules de phytoplancton vivantes d'un type de référence dans l'espace d'essai en fonction de la fluorescence variable déterminée (Fv) sur la base d'un étalonnage linéaire et dans lequel il se fait un calcul d'une quantité équivalente de cellules de phytoplancton vivantes d'autres grandeurs cellulaires que le type de référence au moyen d'une comparaison volumétrique, dans lequel il est prévu une unité de commande au moyen de laquelle on peut commander un dispositif d'évacuation et/ou de désinfection, dans lequel on branche sur le dispositif en amont ou en aval un dispositif d'évacuation et/ou de désinfection, qui reçoit ses ordres de commande de l'unité de commande.

18. Dispositif selon la revendication 17, **caractérisé en ce que** l'espace d'essai est formé d'une cuvette, en particulier constituée de verre ou d'une matière plastique.

19. Dispositif selon la revendication 17 ou la revendication 18, **caractérisé en ce que** l'espace d'essai présente une entrée et/ou une sortie.

20. Dispositif selon l'une quelconque des revendications 17 à 19, **caractérisé en ce qu'**il présente au moins une source de lumière pulsée et/ou au moins une source de lumière continue, des DEL servant en particulier de source de lumière.

21. Dispositif selon l'une quelconque des revendications 17 à 20, **caractérisé en ce qu'**au moins une source de lumière pulsée, en particulier de la lumière bleue d'une longueur d'onde d'environ 420 nm, rouge d'une longueur d'onde d'environ 660 nm, et/ou une source de lumière d'une longueur d'onde de plus de 700 nm sont aménagées.

22. Dispositif selon l'une quelconque des revendications 17 à 21, **caractérisé en ce qu'**au moins une soupape susceptible d'être commandée est aménagée à une entrée et/ou à une sortie de l'espace d'essai.

23. Dispositif selon l'une quelconque des revendications 17 à 22, **caractérisé en ce qu'**il est prévu une pompe d'alimentation pour acheminer l'eau.

24. Dispositif selon l'une quelconque des revendications 17 à 23, **caractérisé en ce qu'**il est prévu une unité de commande, avec laquelle l'unité d'évaluation et/ou une ou plusieurs soupapes et/ou une pompe d'alimentation peut ou peuvent être commandées.

25. Dispositif selon l'une quelconque des revendications 17 à 24, **caractérisé en ce qu'**il prévu une unité d'enregistrement, avec laquelle la ou les fluorescences mesurées (Fo, Fm) et/ou la fluorescence variable déterminée (Fv) et/ou le nombre calculé de cellules de phytoplancton vivantes peut ou peuvent être enregistrées de manière passagère ou continue.
